(19) Europäisches Patentamt
European Patent Office
Office européen des brevets

(11) **EP 2 911 643 B1**

(12) **EUROPEAN PATENT SPECIFICATION**

(45) Date of publication and mention
of the grant of the patent:
**05.12.2018 Bulletin 2018/49**

(21) Application number: **13782691.3**

(22) Date of filing: **22.10.2013**

(51) Int Cl.:
*A61K 8/11* (2006.01)  *B01J 13/16* (2006.01)
*C11D 3/00* (2006.01)  *C11D 3/37* (2006.01)
*C11D 3/50* (2006.01)  *C11D 17/00* (2006.01)
*A61Q 13/00* (2006.01)  *A61K 8/88* (2006.01)
*C11B 9/00* (2006.01)  *C11D 3/20* (2006.01)

(86) International application number:
**PCT/EP2013/072102**

(87) International publication number:
**WO 2014/064122 (01.05.2014 Gazette 2014/18)**

(54) **IMPROVEMENTS RELATING TO ENCAPSULATED BENEFIT AGENTS**

VERBESSERUNG ZUR VERKAPSELTEN PFLEGEMITTELN

AMÉLIORATIONS SE RAPPORTANT À DES AGENTS DE BIENFAIT ENCAPSULÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.10.2012 PCT/CN2012/083461**

(43) Date of publication of application:
**02.09.2015 Bulletin 2015/36**

(73) Proprietors:
• **Unilever PLC
London, Greater London EC4Y 0DY (GB)**
Designated Contracting States:
**CY GB IE MT**
• **Unilever N.V.
3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**

(72) Inventors:
• **JONES, Craig, Warren
Bebington
Wirral
Merseyside CH63 3JW (GB)**
• **LI, Changxi
Shanghai 200335 (CN)**
• **PAN, Xiaoyun
Shanghai 200335 (CN)**
• **WANG, Jinfang
Shanghai 200335 (CN)**
• **ZHANG, Yuanyuan
Shanghai 200335 (CN)**

(74) Representative: **James, Helen Sarah
Unilever Patent Group
Colworth House
Sharnbrook
Bedford MK44 1LQ (GB)**

(56) References cited:
**EP-A1- 1 640 063      EP-A1- 2 468 239
WO-A1-2010/105922    WO-A2-2011/161265**

**Description**

Technical Field

The present invention is concerned with the delivery of particles comprising benefit agents and/or deposition aids, to substrates, with processes for the manufacture of said particles and the manufacture and use of formulations comprising the same. It will be specifically described herein with reference to laundry treatment compositions but has other and broader applications.

Background

Many home care and personal care formulations seek to deliver benefit agents to substrates such as textiles, hard surfaces, hair and skin. Encapsulation of the benefit agent in particles has been proposed as a means of enhancing delivery. Encapsulation of perfumes has generated particular interest and activity in recent years.

Leakage of the benefit agent from the encapsulating particle over time is a known problem with many encapsulates. Leakage into the formulation into which the encapsulate particle has been incorporated leads to instability problems as well as performance issues. Performance issues include not only loss of perfume intensity but also loss of timing of perfume delivery.

WO 12/085864 Discloses a population of encapsulates, the encapsulates comprising a shell and a core, said shell comprising a polyamide polymer that forms a wall that encapsulates said core, said core comprising a perfume composition. The perfume composition comprises perfume raw materials having a ClogP of from 2 to 4.5; the encapsulate has a diameter of from 1 to 100 microns and a fracture strength of from 0.1 to 5 MPa.

US4145184 Discloses a laundry detergent composition comprising, a laundry detergent composition comprising: (a) from 2 to 95 percent of a surfactant selected from the group consisting of anionic, nonionic, ampholytic and zwitterionic surfactants, and mixtures thereof; and (b) an effective amount of a perfuming agent comprising a perfume encapsulated in water insoluble, friable microcapsules having an average size of from 5 to 300 microns. The microcapsules have a shell wall material of polyamide.

WO 09/047745 Discloses a composition comprising an encapsulate comprising a core comprising a benefit agent and a shell that at least encapsulates said core, said encapsulate further comprising a density balancing agent, said composition being a consumer product. The encapsulate's benefit agent is selected from perfume and shell comprises polyamides.

WO 11/056904 Discloses an encapsulate comprising a) a core, comprising perfume and b) shell comprising polyamides.

WO 10/105922 Discloses particles comprising a waxy solid and a polymeric deposition aid having no overall cationic charge, wherein the polymeric deposition aid is partially embedded in the waxy solid.

EP1640063 Discloses polyamide microcapsules of mean diameter 0.0001-5 mm produced by (a) preparing an aqueous phase (a1) containing a di- and/or tri-amine (I); (b) preparing an oil phase containing an oil body (b1), dicarboxylic acid chloride (b2) and tricarboxylic acid chloride (b3) and (c) contacting (a) and (b) to form an emulsion so that polycondensation occurs at the phase boundary.

WO 11/161265 Discloses polyurea and polyamide capsules containing fragrance oils, wherein the oils contain precursors of fragrant aldehydes that are adapted to release the aldehydes under activating conditions.

We have now determined that improved particles comprise a shell which comprises a polyamide (which comprises an aromatic group)and a core comprising a benefit agent and an optional deposition aid. The benefit agent comprises 70-100 wt %, by total weight of the benefit agent, of a component selected from tertiary alcohols, alpha substituted aldehydes, aliphatic and aromatic ketones and ketenes and mixtures thereof, excluding cyclic aliphatic materials containing polar functional groups; further, the perfume is substantially free from aliphatic primary alcohols and aromatic primary alcohols and contains less than 15 wt %, i.e. from 0 to < 15 wt %, preferably from 0 to 14 wt % of aliphatic aldehydes having a chain length of from 8 to 22, by total weight of the benefit agent. Surprisingly, by using these new particles, leakage is much reduced or avoided altogether and perfume is efficiently and timely delivered.

Brief Description of the Invention

Accordingly, in a first aspect the present invention provides a particle comprising:

(a) a core comprising a benefit agent;

(b) a shell, wherein the shell comprises a polyamide, and wherein the polyamide comprises an aromatic group; and

(c) an optional deposition aid;

wherein the benefit agent comprises 70-100 wt %, by total weight of the benefit agent, of a component selected from tertiary alcohols, alpha substituted aldehydes, aliphatic and aromatic ketones and ketenes and mixtures thereof, excluding cyclic aliphatic materials containing polar functional groups;
and the benefit agent is substantially free from i) aliphatic primary alcohols and ii) aromatic primary alcohols and contains less than 15 wt % of aliphatic aldehydes having a chain length of from 8 to 22, by total weight of the benefit agent.

A second aspect of the present invention provides a home care or personal care composition comprising at least one particle according to the first aspect of the invention, the composition preferably being a laundry detergent, laundry conditioner, deodorant, antiperspirant, shampoo, hair conditioner or skin care or skin cleansing product.

A third aspect of the present invention provides a method of treatment of a substrate, preferably wherein the substrate is selected from skin, hair and/or textile material, which includes the step of treating the substrate with a composition comprising particles according to the first aspect of the invention.

Detailed Description of the Invention

In order that the present invention may be further and better understood it will be further described below with reference to specific embodiments of the invention and further preferred and/or optional features. All amounts quoted are wt % of the total composition unless otherwise stated.

The Particle

The core is typically formed in an inner region of the particle and provides a sink for the benefit agent. The "shell" protects the benefit agent and regulates the flow of benefit agent into and out of the core.

Particle Size

The person of ordinary skill in the art will know how to measure the particle size distribution of the capsules, for example, by utilising a Malvern Mastersizer 2000. Typically, the particle has an average diameter of less than 5-50 micron, preferably from 10 to 40 micron, more preferably from 25 to 35 and most preferably 30 micron.

The Core

The core comprises one or more benefit agent.

Advantageously the benefit agent is a hydrophobic benefit agent, preferably an organoleptic benefit agent, for example a flavour or fragrance (the terms "fragrance" and "perfume" are used interchangeably herein).

The benefit agent comprises from 70 to 100 wt %, preferably 70 to 90 wt %, by total weight of the benefit agent, of a component selected from tertiary alcohols, alpha substituted aldehydes, aliphatic and aromatic ketones and ketenes and mixtures thereof.

Further, the benefit agent is substantially free from i) aliphatic primary alcohols and ii) aromatic primary alcohols and preferably comprises from 0 to 15 wt % of aliphatic aldehydes having a chain length of from 8 to 22, by total weight of the benefit agent.

As used herein, by substantially free is meant from 0 to 0.5 wt %, preferably from 0 to 0.05 wt %, more preferably from 0 to 0.005 wt %, even more preferably from 0 to 0.0005 wt %, and most preferably 0 wt% by total weight of the benefit agent.

Aliphatic aldehydes having a chain length of greater than 12, preferably in the range of from 13 to 18 may be present.

Benefit Agents:

The benefit agent comprises 70-100 wt %, by total weight of the benefit agent, of a component selected from tertiary alcohols, alpha substituted aldehydes, aliphatic and aromatic ketones and ketenes and mixtures thereof, excluding cyclic aliphatic materials containing polar functional groups; and the benefit agent is substantially free from i) aliphatic primary alcohols and ii) aromatic primary alcohols and contains less than 15 wt %, i.e. from 0 to < 15 wt %, preferably from 0 to 14 wt % of aliphatic aldehydes having a chain length of from 8 to 22, by total weight of the benefit agent.

Various benefit agents can be incorporated into the particles. Mixtures of benefit agents may be used. Where the end use of the particles is in connection with a surfactant-containing system, any compatible benefit agent which can provide a benefit to a substrate which is treated with a surfactant composition can be used. Preferred benefit agents are in the laundry field, for example fabric benefit agents, and benefit agents which provide a benefit to a laundry wash and/or rinse medium. In the alternative benefit agents may provide a skin or hair related benefit. Advantages of the particles of the invention in the presence of surfactant are a good retention of the benefit agent on storage of a formulation and controllable release of the benefit agent during and after product usage.

Preferred examples include flavours, fragrances, enzymes, antifoams, fluorescer, shading dyes and/or pigments, conditioning agents (for example water-insoluble quaternary ammonium materials and/or silicones), sunscreens, ceramides, antioxidants, reducing agents, sequestrants, colour care additives, density matching polymers, photo-bleaches, lubricants, unsaturated oils, emollients/moisturiser and antimicrobial agents, and mixtures thereof, most preferred are fragrances and antimicrobial agents.

Preferred antimicrobials include Triclosan™, climbazole, octapyrox, ketoconizole, zinc pyrithione, and quaternary ammonium compounds.

Preferred sunscreens and/or skin lightening agents are vitamin B3 compounds. Suitable vitamin B3 compounds are selected from niacin, niacinamide, nicotinyl alcohol, or derivatives or salts thereof. Other vitamins which act as skin lightening agents can be advantageously included in the skin lightening composition to provide for additional skin lightening effects. These include vitamin B6, vitamin C, vitamin A or their precursors. Mixtures of the vitamins can also be employed in the composition of the invention. An especially preferred additional vitamin is vitamin B6. Other non-limiting examples of skin lightening agents useful herein include adapalene, aloe extract, ammonium lactate, arbutin, azelaic acid, butyl hydroxy anisole, butyl hydroxy toluene, citrate esters, deoxyarbutin, 1,3 diphenyl propane derivatives, 2, 5 di hydroxyl benzoic acid a nd its derivatives, 2-(4-acetoxyphenyl)-1 ,3 d itha ne, 2-(4- Hydroxylphenyl)-1,3 dithane, ellagic acid, gluco pyranosyl-1-ascorbate, gluconic acid, glycolic acid, green tea extract, 4-Hydroxy-5-methyl-3[2H]-furanone, hydroquinone, 4 hydroxyanisole and its derivatives, 4-hydroxy benzoic acid derivatives, hydroxycaprylic acid, inositol ascorbate, kojic acid, lactic acid, lemon extract, linoleic acid, magnesium ascorbyl phosphate, 5-octanoyl salicylic acid, 2,4 resorcinol derivatives, 3,5 resorcinol derivatives, salicylic acid, 3,4,5 trihydroxybenzyl derivatives, and mixtures thereof. Preferred sunscreens useful in the present invention are 2-ethylhexyl-p-methoxycinnamate, butyl methoxy dibenzoylmethane, 2-hydroxy-4-methoxybenzophenone, octyl dimethyl-p-aminobenzoic acid and mixtures thereof. Particularly preferred sunscreen is chosen from 2-ethyl hexyl-p-methoxycinnamate, 4,- t-butyl-4'- methoxydibenzoyl-methane or mixtures thereof. Other conventional sunscreen agents that are suitable for use in the skin lightening composition of the invention include 2-hydroxy-4-methoxybenzophenone, octyldimethyl- p-a mi nobenzoic acid, digalloyltrioleate, 2,2-dihydroxy-4- methoxybenzophenone, ethyl-4-(bis(hydroxypropyl)) aminobenzoate, 2- ethylhexyl-2- cyano-3,3-diphenylacrylate, 2-ethylhexylsalicylate, glyceryl- p-aminobenzoate, 3,3,5- trimethylcyclohexyl-salicylate, methylanthranilate, p-dimethyl-aminobenzoic acid or aminobenzoate, 2-ethylhexyl-p-dimethyl- amino-benzoate, 2-phenylbenzimidazole-5-sulfonic acid, 2-(p- dimethylaminophenyl)-5-sulfonic benzoxazoic acid and mixtures of these compounds.

Preferred anti-oxidants include vitamin E, retinol, antioxiants based on hydroxytoluene such as Irganox™ or commercially available antioxidants such as the Trollox™ series.

Perfume and fragrance materials (which include pro-fragrances) are a particularly preferred benefit agent.

The pro-fragrance can, for example, be a food lipid. Food lipids typically contain structural units with pronounced hydrophobicity. The majority of lipids are derived from fatty acids. In these 'acyl' lipids the fatty acids are predominantly present as esters and include mono-, di-, triacyl glycerols, phospholipids, glycolipids, diol lipids, waxes, sterol esters and tocopherols. In their natural state, plant lipids comprise antioxidants to prevent their oxidation. While these may be at least in part removed during the isolation of oils from plants some antioxidants may remain. These antioxidants can be pro-fragrances. In particular, the carotenoids and related compounds including vitamin A, retinol, retinal, retinoic acid and provitamin A are capable of being converted into fragrant species including the ionones, damascones and damscenones. Preferred pro-fragrance food lipids include olive oil, palm oil, canola oil, squalene, sunflower seed oil, wheat germ oil, almond oil, coconut oil, grape seed oil, rapeseed oil, castor oil, corn oil, cottonseed oil, safflower oil, groundnut oil, poppy seed oil, palm kernel oil, rice bran oil, sesame oil, soybean oil, pumpkin seed oil, jojoba oil and mustard seed oil. Perfume components which are odiferous materials are described in further detail below.

The perfume is typically present in an amount of from 10-85% by total weight of the particle, preferably from 15 to 75% by total weight of the particle. The perfume suitably has a molecular weight of from 50 to 500Dalton. Pro-fragrances can be of higher molecular weight, being typically 1-10 kD.

Useful components of the perfume include materials of both natural and synthetic origin. They include single compounds and mixtures. Specific examples of such components may be found in the current literature, e.g., in Fenaroli's Handbook of Flavour Ingredients, 1975, CRC Press; Synthetic Food Adjuncts, 1947 by M. B. Jacobs, edited by Van Nostrand; or Perfume and Flavour Chemicals by S. Arctander 1969, Montclair, N.J. (USA). These substances are well known to the person skilled in the art of perfuming, flavouring, and/or aromatizing consumer products, i.e., of imparting an odour and/or a flavour or taste to a consumer product traditionally perfumed or flavoured, or of modifying the odour and/or taste of said consumer product.

By perfume in this context is not only meant a fully formulated product fragrance, but also selected components of that fragrance, particularly those which are prone to loss, such as the so-called 'top notes'.

Top notes are defined by Poucher (Journal of the Society of Cosmetic Chemists 6(2):80 [1955]). Examples of well known top-notes include citrus oils, linalool, linalyl acetate, lavender, dihydromyrcenol, rose oxide and cis-3-hexanol. Top notes typically comprise 15-25%wt of a perfume composition and in those embodiments of the invention which contain an increased level of top-notes it is envisaged at that least 20%wt would be present within the particle.

Typical perfume components which it is advantageous to employ in the embodiments of the present invention include those with a relatively low boiling point, preferably those with a boiling point of less than 300, preferably 100-250 Celsius.

It is also advantageous to encapsulate perfume components which have a low LogP (i.e. those which will be partitioned into water), preferably with a LogP of less than 3.0. These materials, of relatively low boiling point and relatively low LogP have been called the "delayed blooming" perfume ingredients and include the following materials:

Allyl Caproate, Amyl Acetate, Amyl Propionate, Anisic Aldehyde, Anisole, Benzaldehyde, Benzyl Acetate, Benzyl Acetone, Benzyl Alcohol, Benzyl Formate, Benzyl Iso Valerate, Benzyl Propionate, Beta Gamma Hexenol, Camphor Gum, Laevo-Carvone, d-Carvone, Cinnamic Alcohol, Cinamyl Formate, Cis-Jasmone, cis-3-Hexenyl Acetate, Cuminic Alcohol, Cyclal C, Dimethyl Benzyl Carbinol, Dimethyl Benzyl Carbinol Acetate, Ethyl Acetate, Ethyl Aceto Acetate, Ethyl Amyl Ketone, Ethyl Benzoate, Ethyl Butyrate, Ethyl Hexyl Ketone, Ethyl Phenyl Acetate, Eucalyptol, Eugenol, Fenchyl Acetate, Flor Acetate (tricyclo Decenyl Acetate), Frutene (tricyclco Decenyl Propionate), Geraniol, Hexenol, Hexenyl Acetate, Hexyl Acetate, Hexyl Formate, Hydratropic Alcohol, Hydroxycitronellal, Indone, Isoamyl Alcohol, Iso Menthone, Isopulegyl Acetate, Isoquinolone, Ligustral, Linalool, Linalool Oxide, Linalyl Formate, Menthone, Menthyl Acetphenone, Methyl Amyl Ketone, Methyl Anthranilate, Methyl Benzoate, Methyl Benyl Acetate, Methyl Eugenol, Methyl Heptenone, Methyl Heptine Carbonate, Methyl Heptyl Ketone, Methyl Hexyl Ketone, Methyl Phenyl Carbinyl Acetate, Methyl Salicylate, Methyl-N-Methyl Anthranilate, Nerol, Octalactone, Octyl Alcohol, p-Cresol, p-Cresol Methyl Ether, p-Methoxy Acetophenone, p-Methyl Acetophenone, Phenoxy Ethanol, Phenyl Acetaldehyde, Phenyl Ethyl Acetate, Phenyl Ethyl Alcohol, Phenyl Ethyl Dimethyl Carbinol, Prenyl Acetate, Propyl Bornate, Pulegone, Rose Oxide, Safrole, 4-Terpinenol, Alpha-Terpinenol, and /or Viridine.

It is commonplace for a plurality of perfume components to be present in a formulation. In the encapsulates of the present invention it is envisaged that there may be four or more, preferably five or more, more preferably six or more or even seven or more different perfume components from the list given of delayed blooming perfumes given above present in the particles.

Another group of perfumes with which the present invention can be applied are the so-called 'aromatherapy' materials. These include many components also used in perfumery, including components of essential oils such as Clary Sage, Eucalyptus, Geranium, Lavender, Mace Extract, Neroli, Nutmeg, Spearmint, Sweet Violet Leaf and Valerian. By means of the present invention these materials can be transferred to textile articles that will be worn or otherwise come into contact with the human body (such as handkerchiefs and bed linen).

The Shell

The shell comprises a polyamide, which comprises an aromatic group.

The polyamide polymer may comprise at least one water miscible monomer and one water immiscible organic monomer.

The water miscible monomer may comprise a material selected from the group consisting of a diamine, a triamine and mixtures thereof. The diamines and triamines themselves may be selected from the group consisting of diethylene triamine, hexamethylene diamine, ethylene diamine and mixtures thereof. The water immiscible organic monomer may be selected from the group consisting of diacyl chlorides, triacyl chlorides and mixtures thereof. The diacyl chlorides may be selected from the group consisting of sebacoyl dichloride, adipoyl dichloride, and mixtures thereof and said triacyl chlorides may be selected from the group consisting of teraphthaloyl chloride, trimesoyl chloride, acetyl chloride, benzoyl chloride, 1, 3, 5-benzentricarbonyl chloride, and mixtures thereof.

In one embodiment, said polyamide polymer may comprise two or more water miscible monomers.

Deposition Aids:

The particle preferably comprises a deposition aid. In particularly preferred embodiments the deposition aid is substantive to proteinaceous, cellulosic, polyester, lipid or polyamide surfaces. By use of such a deposition aid, the efficiency of delivery to a specific substrate may be enhanced.

Deposition aids modify the properties of the exterior of the particle. One particular benefit which can be obtained with these materials is to make the particle more substantive to a desired substrate. Desired substrates include cellulosics (including cotton), polyesters (including those employed in the manufacture of polyester fabrics) and protein-containing substrates (such as akin and hair). Deposition aids are preferably selected from non-hydrolysable substrate-substantive polymers, hydrolysable substrate-substantive polymers and polyester-substantive polymers.

Preferred polysaccharide polymers, whether hydrolysable or not may be derived from a broad range of polysaccharides. Preferably, the polysaccharide is selected from the group consisting of: tamarind gum (preferably consisting of xyloglucan polymers), guar gum, locust bean gum (preferably consisting of galactomannan polymers), and other industrial gums and polymers, which include, but are not limited to, Tara, Fenugreek, Aloe, Chia, Flaxseed, Psyllium seed, quince seed, xanthan, gellan, welan, rhamsan, dextran, curdlan, pullulan, scleroglucan, schizophyllan, chitin, hydroxyalkyl cellulose, arabinan (preferably from sugar beets), de- branched arabinan (preferably from sugar beets), arabinoxylan (preferably from rye and wheat flour), galactan (preferably from lupin and potatoes), pectic galactan (preferably from potatoes), galactomannan (preferably from carob, and including both low and high viscosities), glucomannan, lichenan (preferably from icelandic moss), mannan (preferably from ivory nuts), pachyman, rhamnogalacturonan, acacia gum, agar, alginates, carrageenan, chitosan, clavan, hyaluronic acid, heparin, inulin, cellodextrins, cellulose, cellulose derivatives and mixtures thereof.

Preferred non-hydrolysable substrate-substantive deposition aids include non-hydrolysable polysaccharides. The polysaccharide preferred for cotton substantivity for example has a $\beta$-1,4-linked backbone.

Preferably the polysaccharide is a cellulose, a cellulose derivative, or another $\beta$-1,4-linked polysaccharide having an affinity for cellulose, such as polymannan, polyglucan, polyglucomannan, polyxyloglucan and polygalactomannan or a mixture thereof. More preferably, the polysaccharide is selected from the group consisting of polyxyloglucan and polygalactomannan. Most preferably, the deposition aid is locust bean gum, xyloglucan, guar gum or mixtures thereof.

Preferred hydrolysable substrate-substantive deposition aids include hydrolysable polysaccharides. These comprise a polysaccharide which has been modified to render it more water soluble by means of a group covalently attached to the polysaccharide by means of hydrolysable bond. Preferred groups may for example be independently selected from one or more of acetate, propanoate, trifluoroacetate, 2- (2-hydroxy-1-oxopropoxy) propanoate, lactate, glycolate, pyruvate, crotonate, isovalerate cinnamate, formate, salicylate, carbamate, methylcarbamate, benzoate, gluconate, methanesulphonate, toluene, sulphonate, groups and hemiester groups of fumaric, malonic, itaconic, oxalic, maleic, succinic, tartaric, aspartic, glutamic, and malic acids.

Preferred amongst such hydrolysable deposition aids for cotton substantivity is cellulose mono acetate.

Suitable and preferred polyester-substantive deposition aids include phthalate containing polymers, more preferably a polymer having one or more nonionic hydrophilic components comprising oxyethylene, polyoxyethylene, oxypropylene or polyoxypropylene segments, and, one or more hydrophobic components comprising terephthalate segments. Typically, oxyalkylene segments of these deposition aids will have a degree of polymerization of from 1 to about 400, although higher levels can be used, preferably from 100 to about 350, more preferably from 200 to about 300.

One type of preferred deposition aid is a copolymer having random blocks of ethylene terephthalate and polyethylene oxide terephthalate.

Another preferred polymeric deposition aid is polyester with repeat units of ethylene terephthalate units contains 10-15% by weight of ethylene terephthalate units together with 90-80% by weight of polyoxyethylene terephthalate units, derived from a polyethylene glycol of average molecular weight 0.2kD-40kD. Examples of this class of polymer include the commercially available material ZELCON 5126 (from DuPont) and MILEASE T (from ICI). Examples of related polymers can be found in US 4702857.

Another preferred polymeric deposition aid is a sulfonated product of a substantially linear ester oligomer comprised of an oligomeric ester backbone of terephthaloyl and oxyalkyleneoxy repeat units and terminal moieties covalently attached to the backbone. These soil release agents are described fully in US 4968451. Other suitable polymeric soil release agents include the terephthalate polyesters of US 4711730, the anionic end-capped oligomeric esters of US 4721580, and the block polyester oligomeric compounds of US 4702857.

Preferred polymeric deposition aids also include the soil release agents of U.S. 4877896 which discloses anionic, especially sulfoarolyl, end-capped terephthalate esters.

Still another preferred deposition aid is an oligomer with repeat units of terephthaloyl units, sulfoisoterephthaloyl units, oxyethyleneoxy and oxy-1,2-propylene units. The repeat units form the backbone of the oligomer and are preferably terminated with modified isethionate end-caps. A particularly preferred deposition aid of this type comprises about one sulfoisophthaloyl unit, 5 terephthaloyl units, oxyethyleneoxy and oxy-1,2-propyleneoxy units in a ratio of from about 1.7 to about 1.8, and two end-cap units of sodium 2-(2-hydroxyethoxy)-ethanesulfonate. Said soil release agent also comprises from about 0.5% to about 20%, by weight of the oligomer, of a crystalline-reducing stabilizer, preferably selected from the group consisting of xylene sulfonate, cumene sulfonate, toluene sulfonate, and mixtures thereof.

The deposition aid may be straight or branched. The preferred molecular weight of the polymeric deposition aid is in the range of from about 5kD to about 500kD, preferably 10kD-500kD, more preferably 20kD-300kD.

Preferably, the deposition-aid polymer is present at levels such that the ratio polymer:particle solids is in the range 1:500-3:1, preferably 1:200-1:3.

Preparation Methods:

The benefit agent may be present in the reaction mixture, at a level to give the benefit agent levels in the resulting particles at the levels disclosed above, although it is also possible to form "empty" particles (with or without a benefit agent carrier, for example wax) and subsequently expose them to a benefit agent which can be adsorbed into the inner region.

Deposition aids are generally added to the aqueous phase towards the end of the process, where, for example, further monomer(s) can be added to form further shell material and bind additional materials to the outside of the particle.

Deposition aid may added at the end of the later phase (preferably after cooling), when for example, further shell forming material (for example further isocyanate and co-momomer) are also added to bind the deposition aid to the outer surface of the particle by the formation of further shell material which entraps a portion of the deposition aid and leads to a "hairy" particle in which the "hair" comprises the deposition aid.

For simple core-shell particles, the core excluding benefit agent is less than or equal to 95%wt of mass, and the shell generally 5%wt or greater of the mass of the particle.

Particularly Preferred Embodiments:

It is particularly preferred that the above particle comprises a fragrance contained in the core, surrounded by a shell and/or adsorbed into a carrier material, for example a mineral oil, that is surrounded by the shell and/or a polysaccharide deposition aid exterior to the shell. Especially preferred particles have a particle size of 5-50 microns.

Use in Products:

The end-product compositions of the invention may be in any physical form e.g. a solid such as a powder or granules, a tablet, a solid bar, a paste, gel or liquid, especially, an aqueous-based liquid.

The particles of the invention may be advantageously incorporated into surfactant-containing and, in particular laundry and personal care compositions. The particles are typically included in said compositions at levels of from 0.001% to 10%, preferably from 0.005% to 7.55%, most preferably from 0.01% to 5% by weight of the total composition.

For laundry applications, one active ingredient in the compositions is preferably a surface active agent or a fabric conditioning agent. More than one active ingredient may be included. For some applications a mixture of active ingredients may be used.

Formulated compositions comprising the particles of the invention may contain a surface-active compound (surfactant) which may be chosen from soap and non soap anionic, cationic, non-ionic, amphoteric and zwitterionic surface active compounds and mixtures thereof. Many suitable surface active compounds are available and are fully described in the literature, for example, in "Surface-Active Agents and Detergents", Volumes I and II, by Schwartz, Perry and Berch. The preferred surface-active compounds that can be used are soaps and synthetic non soap anionic, and non-ionic compounds.

The compositions of the invention may contain linear alkylbenzene sulphonate, particularly linear alkylbenzene sulphonates having an alkyl chain length of from C8 to C15. It is preferred if the level of linear alkylbenzene sulphonate is from 0 wt% to 30 wt%, more preferably from 1 wt% to 25 wt%, most preferably from 2 wt% to 15 wt%, by weight of the total composition.

Compositions may contain other anionic surfactants in amounts additional to the percentages quoted above. Suitable anionic surfactants are well-known to those skilled in the art. Examples include primary and secondary alkyl sulphates, particularly C8 to C15 primary alkyl sulphates; alkyl ether sulphates; olefin sulphonates; alkyl xylene sulphonates; dialkyl sulphosuccinates; and fatty acid ester sulphonates. Sodium salts are generally preferred.

Compositions may also contain non-ionic surfactant. Nonionic surfactants that may be used include the primary and secondary alcohol ethoxylates, especially the C8 to C20 aliphatic alcohols ethoxylated with an average of from 1 to 20 moles of ethylene oxide per mole of alcohol, and more especially the C10 to CI5 primary and secondary aliphatic alcohols ethoxylated with an average of from 1 to 10 moles of ethylene oxide per mole of alcohol. Non ethoxylated nonionic surfactants include alkylpolyglycosides, glycerol monoethers, and polyhydroxyamides (glucamide).

It is preferred if the level of non-ionic surfactant is from 0 wt% to 30 wt%, preferably from 1 wt% to 25 wt%, most preferably from 2 wt% to 15 wt%, by weight of a fully formulated composition comprising the particles of the invention.

Any conventional fabric conditioning agent may be used. The conditioning agents may be cationic or non-ionic. If the fabric conditioning compound is to be employed in a main wash detergent composition the compound will typically be non-ionic. For use in the rinse phase, typically they will be cationic. They may for example be used in amounts from 0.5% to 35%, preferably from 1% to 30% more preferably from 3% to 25% by weight of a fully formulated composition comprising the particles of the invention.

Suitable cationic fabric softening compounds are substantially water-insoluble quaternary ammonium materials comprising a single alkyl or alkenyl long chain having an average chain length greater than or equal to C20 or, more preferably, compounds comprising a polar head group and two alkyl or alkenyl chains having an average chain length greater than or equal to C14. Preferably the fabric softening compounds have two long chain alkyl or alkenyl chains each having an average chain length greater than or equal to C16. Most preferably at least 50% of the long chain alkyl or alkenyl groups have a chain length of C18 or above. It is preferred if the long chain alkyl or alkenyl groups of the fabric softening compound are predominantly linear.

Quaternary ammonium compounds having two long-chain aliphatic groups, for example, distearyldimethyl ammonium chloride and di(hardened tallow alkyl) dimethyl ammonium chloride, are widely used in commercially available rinse conditioner compositions. Other examples of these cationic compounds are to be found in "Surfactants Science Series" volume 34 ed. Richmond 1990, volume 37 ed. Rubingh 1991 and volume 53 eds. Cross and Singer 1994, Marcel Dekker Inc. New York".

The fabric softening compounds are preferably compounds that provide excellent softening, and are characterised by a chain melting L• to L• transition temperature greater than 25 Celsius, preferably greater than 35 Celsius, most preferably greater than 45 Celsius. This L• to L• transition can be measured by differential scanning calorimetry as defined in "Handbook of Lipid Bilayers", D Marsh, CRC Press, Boca Raton, Florida, 1990 (pages 137 and 337).

Substantially water-insoluble fabric softening compounds are defined as fabric softening compounds having a solubility of less than $1 \times 10^{-3}$ wt% in demineralised water at 20 Celsius. Preferably the fabric softening compounds have a solubility of less than $1 \times 10^{-4}$ wt%, more preferably from less than $1 \times 10^{-8}$ to $1 \times 10^{-6}$ wt%.

Especially preferred are cationic fabric softening compounds that are water-insoluble quaternary ammonium materials having two C12-22 alkyl or alkenyl groups connected to the molecule via at least one ester link, preferably two ester links. Di(tallowoxyloxyethyl) dimethyl ammonium chloride and/or its hardened tallow analogue is an especially preferred compound of this class.

A second preferred type comprises those derived from triethanolamine (hereinafter referred to as 'TEA quats') as described in for example US 3915867. Suitable materials are, for example, N-methyl-N,N,N-triethanolamine ditallowester or di-hardened-tallowester quaternary ammonium chloride or methosulphate.

Examples of commercially available TEA quats include Rewoquat WE18 and Rewoquat WE20, both partially unsaturated (ex. WITCO), Tetranyl AOT-1, fully saturated (ex. KAO) and Stepantex VP 85, fully saturated (ex. Stepan).

It is advantageous if the quaternary ammonium material is biologically biodegradable.

It is also possible to include certain mono-alkyl cationic surfactants which can be used in main-wash compositions for fabrics. Cationic surfactants that may be used include quaternary ammonium salts of the general formula R1R2R3R4N+ X-wherein the R groups are long or short hydrocarbon chains, typically alkyl, hydroxyalkyl or ethoxylated alkyl groups, and X is a counter-ion (for example, compounds in which R1 is a C8-C22 alkyl group, preferably a C8-C10 or C12-C14 alkyl group, R2 is a methyl group, and R3 and R4, which may be the same or different, are methyl or hydroxyethyl groups); and cationic esters (for example, choline esters).

The choice of surface-active compound (surfactant), and the amount present, will depend on the intended use of the detergent composition. In fabric washing compositions, different surfactant systems may be chosen, as is well known to the skilled formulator, for hand-washing products and for products intended for use in different types of washing machine.

The total amount of surfactant present will also depend on the intended end use and may, in fully formulated products, be as high as 60 wt%, for example, in a composition for washing fabrics by hand. In compositions for machine washing of fabrics, an amount of from 5 to 40 wt% is generally appropriate. Typically compositions will comprise at least 2 wt% surfactant e.g. 2-60%, preferably 15-40% most preferably 25-35%, by weight.

Detergent compositions suitable for use in most automatic fabric washing machines generally contain anionic non-soap surfactant, or non-ionic surfactant, or combinations of the two in any suitable ratio, optionally together with soap. Compositions, when used as main wash fabric washing compositions, will generally also contain one or more detergency builders. The total amount of detergency builder in compositions will typically range from 5 to 80 wt%, preferably from 10 to 60 wt%, by weight of composition.

Inorganic builders that may be present include sodium carbonate, if desired in combination with a crystallisation seed for calcium carbonate, as disclosed in GB 1 437 950 (Unilever); crystalline and amorphous aluminosilicates, for example, zeolites as disclosed in GB 1 473 201 (Henkel), amorphous aluminosilicates as disclosed in GB 1 473 202 (Henkel) and mixed crystalline/amorphous aluminosilicates as disclosed in GB 1 470 250 (Procter & Gamble); and layered silicates as disclosed in EP 164 514B (Hoechst). Inorganic phosphate builders, for example, sodium orthophosphate, pyrophosphate and tripolyphosphate are also suitable for use with this invention.

The compositions of the invention preferably contain an alkali metal, preferably sodium, aluminosilicate builder. Sodium aluminosilicates may generally be incorporated in end product formulations amounts of from 10 to 70% by weight (anhydrous basis), preferably from 25 to 50 wt%.

The alkali metal aluminosilicate may be either crystalline or amorphous or mixtures thereof, having the general formula: $0.8\ 1.5\ Na_2O.\ Al_2O_3.\ 0.8\ 6\ SiO_2$

These materials contain some bound water and are required to have a calcium ion exchange capacity of at least 50 mg CaO/g. The preferred sodium aluminosilicates contain 1.5 3.5 SiO2 units (in the formula above). Both the amorphous and the crystalline materials can be prepared readily by reaction between sodium silicate and sodium aluminate, as amply described in the literature. Suitable crystalline sodium aluminosilicate ion exchange detergency builders are described, for example, in GB 1 429 143 (Procter & Gamble). The preferred sodium aluminosilicates of this type are the well known commercially available zeolites A and X, and mixtures thereof.

The zeolite may be the commercially available zeolite 4A now widely used in laundry detergent powders. However, according to a preferred embodiment of the invention, the zeolite builder incorporated in the compositions of the invention is maximum aluminium zeolite P (zeolite MAP) as described and claimed in EP 384 070A (Unilever). Zeolite MAP is defined as an alkali metal aluminosilicate of the zeolite P type having a silicon to aluminium weight ratio not exceeding 1.33, preferably within the range of from 0.90 to 1.33, and more preferably within the range of from 0.90 to 1.20.

Especially preferred is zeolite MAP having a silicon to aluminium weight ratio not exceeding 1.07, more preferably about 1.00. The calcium binding capacity of zeolite MAP is generally at least 150 mg CaO per g of anhydrous material.

Organic builders that may be present include polycarboxylate polymers such as polyacrylates, acrylic/maleic copolymers, and acrylic phosphinates; monomeric polycarboxylates such as citrates, gluconates, oxydisuccinates, glycerol mono , di¬ and trisuccinates, carboxymethyloxy succinates, carboxymethyloxymalonates, dipicolinates, hydroxyethyliminodiacetates, alkyl and alkenylmalonates and succinates; and sulphonated fatty acid salts. This list is not intended to be exhaustive.

Especially preferred organic builders are citrates, suitably used in fully formulated compositions in amounts of from 5 to 30 wt%, preferably from 10 to 25 wt%; and acrylic polymers, more especially acrylic/maleic copolymers, suitably used in amounts of from 0.5 to 15 wt%, preferably from 1 to 10 wt%.

Builders, both inorganic and organic, are preferably present in alkali metal salt, especially sodium salt, form.

Compositions comprising particles according to the invention may also suitably contain a bleach system. Fabric washing compositions may desirably contain peroxy bleach compounds, for example, inorganic persalts or organic peroxyacids, capable of yielding hydrogen peroxide in aqueous solution.

Suitable peroxy bleach compounds include organic peroxides such as urea peroxide, and inorganic persalts such as the alkali metal perborates, percarbonates, perphosphates, persilicates and persulphates. Preferred inorganic persalts are sodium perborate monohydrate and tetrahydrate, and sodium percarbonate.

Especially preferred is sodium percarbonate having a protective coating against destabilisation by moisture. Sodium percarbonate having a protective coating comprising sodium metaborate and sodium silicate is disclosed in GB 2 123 044B (Kao).

The peroxy bleach compound is suitably present in a fully formulated product in an amount of from 0.1 to 35 wt%, preferably from 0.5 to 25 wt%. The peroxy bleach compound may be used in conjunction with a bleach activator (bleach precursor) to improve bleaching action at low wash temperatures. The bleach precursor is suitably present in an amount of from 0.1 to 8 wt%, preferably from 0.5 to 5 wt%.

Preferred bleach precursors are peroxycarboxylic acid precursors, more especially peracetic acid precursors and pernoanoic acid precursors. Especially preferred bleach precursors suitable for use in the present invention are N,N,N',N' tetracetyl ethylenediamine (TAED) and sodium nonanoyloxybenzene sulphonate (SNOBS). The novel quaternary ammonium and phosphonium bleach precursors disclosed in US 4 751 015 and US 4 818 426 (Lever Brothers Company) and EP 402 971A (Unilever), and the cationic bleach precursors disclosed in EP 284 292A and EP 303 520A (Kao) are also of interest.

The bleach system can be either supplemented with or replaced by a peroxyacid. Examples of such peracids can be found in US 4 686 063 and US 5 397 501 (Unilever). A preferred example is the imido peroxycarboxylic class of peracids described in EP A 325 288, EP A 349 940, DE 382 3172 and EP 325 289. A particularly preferred example is phthalimido peroxy caproic acid (PAP). Such peracids are suitably present at 0.1-12%wt, preferably 0.5-10%wt.

A bleach stabiliser (transition metal sequestrant) may also be present in fully formulated products. Suitable bleach stabilisers include ethylenediamine tetraacetate (EDTA), the polyphosphonates such as Dequest (Trade Mark) and non phosphate stabilisers such as EDDS (ethylene diamine di succinic acid). These bleach stabilisers are also useful for stain removal especially in end-products containing low levels of bleaching species or no bleaching species.

An especially preferred bleach system comprises a peroxy bleach compound (preferably sodium percarbonate optionally together with a bleach activator), and a transition metal bleach catalyst as described and claimed in EP 458 397A, EP 458 398A and EP 509 787A (Unilever).

Advantageously in the compositions of the invention benefit agents, particularly, perfume components may be employed which are sensitive to bleaches as the encapsulation of, for example, the perfume component within the particles will provide some degree of protection to the perfume component or other benefit agent.

The fully formulated compositions may also contain one or more enzyme(s).

Suitable enzymes include the proteases, amylases, cellulases, oxidases, peroxidases and lipases usable for incorporation in detergent compositions. Preferred proteolytic enzymes (proteases) are, catalytically active protein materials which degrade or alter protein types of stains when present as in fabric stains in a hydrolysis reaction. They may be of any suitable origin, such as vegetable, animal, bacterial or yeast origin.

Proteolytic enzymes or proteases of various qualities and origins and having activity in various pH ranges of from 4-12 are available and can be used in the instant invention. Examples of suitable proteolytic enzymes are the subtilisins which are obtained from particular strains of B. Subtilis B. licheniformis, such as the commercially available subtilisins Maxatase (Trade Mark), as supplied by Genencor International N.V., Delft, Holland, and Alcalase (Trade Mark), as supplied by Novozymes Industri A/S, Copenhagen, Denmark.

Particularly suitable is a protease obtained from a strain of Bacillus having maximum activity throughout the pH range of 8-12, being commercially available, e.g. from Novozymes Industri A/S under the registered trade names Esperase (Trade Mark) and Savinase (Trade Mark). The preparation of these and analogous enzymes is described in GB 1 243 785. Other commercial proteases are Kazusase (Trade Mark obtainable from Showa Denko of Japan), Optimase (Trade Mark from Miles Kali Chemie, Hannover, West Germany), and Superase (Trade Mark obtainable from Pfizer of U.S.A.).

Detergency enzymes are commonly employed in fully formulated products in granular form in amounts of from about 0.1 to about 3.0 wt% on product. However, any suitable physical form of enzyme may be used. Advantageously in the compositions of the invention benefit agents, for example, perfume components, may be employed which are sensitive to enzymes as the encapsulation of the perfume component (or other benefit agent) within the particles will provide some degree of protection to the perfume component (or other benefit agent).

The compositions of the invention may contain alkali metal, preferably sodium carbonate, in order to increase detergency and ease processing. Sodium carbonate may suitably be present in fully formulated products in amounts ranging from 1 to 60 wt%, preferably from 2 to 40 wt%. However, compositions containing little or no sodium carbonate are also within the scope of the invention.

The fully formulated detergent composition when diluted in the wash liquor (during a typical wash cycle) will typically give a pH of the wash liquor from 7 to 10.5 for a main wash detergent.

Particulate detergent compositions are suitably prepared by spray drying a slurry of compatible heat insensitive ingredients, and then spraying on or post-dosing those ingredients unsuitable for processing via the slurry. The skilled detergent formulator will have no difficulty in deciding which ingredients should be included in the slurry and which should not. It is particularly useful to add the perfume particles of the present invention via post-dosing.

Particulate detergent compositions preferably have a bulk density of at least 400 g/litre, more preferably at least 500 g/litre. Especially preferred compositions have bulk densities of at least 650 g/litre, more preferably at least 700 g/litre.

Such powders may be prepared either by post tower densification of spray dried powder, or by wholly non tower methods such as dry mixing and granulation; in both cases a high-speed mixer/granulator may advantageously be used. Processes using high speed mixer/granulators are disclosed, for example, in EP 340 013A, EP 367 339A, EP 390 251A and EP 420 317A (Unilever).

Liquid detergent compositions can be prepared by admixing the essential and optional ingredients thereof in any desired order to provide compositions containing components in the requisite concentrations. Liquid compositions according to the present invention can also be in compact form which means it will contain a lower level of water compared to a conventional liquid detergent.

In order that the present invention may be still further understood and carried forth into practice it will be further described with reference to the following examples:

## Examples

## Materials Used

Raw materials used in the following examples are summarised in Table 1.

**Table 1: Name, supplier and description of materials used in these examples.**

| Material | Supplier | Description/Function |
|---|---|---|
| Diethylenetriamine (DETA) | Alfa Acesar | Reactive amine to form the polyamide shell. |
| Terephthaloyl chloride | Aldrich | Reactive acid chloride to form the polyamide shell. |
| PVA (5-88) | Kuraray | Poly(vinylalcolhol) Colloid stabilizer |

(continued)

| Material | Supplier | Description/Function |
|---|---|---|
| Dodecylbenzenesulphonic acid sodium salt (LAS) | Aldrich | Anionic surfactant |
| Synperonic A7 | Uniqema | Fatty alcohol ethoxylate, nonionic surfactant |
| Sodium carbonate | Shanghai Lingfeng Chemical Reagent Co., Ltd | pH regulator |
| Sodium bicarbonate | Shanghai Hongguang Co., Ltd. | pH regulator |

Model perfumes were prepared for use in the following examples, the compositions of which are summarised in Table 2.

Table 2: Composition of model perfumes, showing ingredient, supplier and amount.

| Ingredient | Amount (wt % of total perfume composition) | Supplier |
|---|---|---|
| **Perfume X** | | - |
| Linalool | 60% | Fluka |
| Benzyl acetate | 30% | TCI |
| Limonene | 10% | TCI |
| **Perfume Y[1]** | | Fragrance Oils International Ltd. |
| Linalyl acetate | 6.7 | - |
| OTBCA[2] | 11.3 | - |
| Cyclopentadecanolid | 6.7 | - |
| Manzanate | 6.7 | - |
| Octanol | 6.7 | - |
| Tetra hydro Linalool | 6.7 | - |
| Benzyl acetate | 6.7 | - |
| Damascone, delta | 1.6 | - |
| Dodecylaldehyde | 6.7 | - |
| Verdyl acetate | 6.7 | - |
| Ionone beta | 6.7 | - |
| Bangalol | 6.7 | - |
| Iso E super (OTNE)[3] | 6.7 | - |
| Hexyl cinnamic aldehyde | 6.7 | - |
| **Further perfume raw materials "Z"** | | |
| Octanal (C8 aliphatic aldehyde) | | |
| Dodecyl aldehyde (C12 aliphatic aldehyde) | | |
| benzyl alcohol (aromatic primary alcohol) | | |

(continued)

| Further perfume raw materials "Z" | | |
|---|---|---|
| Octyl alcohol (aliphatic primary alcohol) | | |

[1]Supplied by Fragrance Oils International Ltd.
[2]Ortho-tertiary-butyl cyclohexyl acetate
[3]1-(1,2,3,4,5,6,7,8-octahydro-2,3,8,8-tetramethyl-2-naphthyl)ethan-1-one

**Example 1:- Preparation of Capsule 1X, Capsule 1Y in accordance with the invention and Capsule AZ**

**Method of preparation of Capsule 1X and Capsule 1Y**

Capsule 1X and Capsule 1Y, comprising perfume X and perfume Y respectively, were prepared using the following method:-

Step 1:- The following liquids were prepared:-

**Liquid A:** 2.4 ml perfume (X or Y) and 0.27 g terephthaloyl chloride were mixed until the terephthaloyl chloride dissolved to obtain an oily liquid.

**Water solution B:** 30 ml deionised water containing 1 wt% PVA (5-88) was prepared and the pH adjusted to desired value using 1M NaOH.

**Water solution C:** 3.9 ml DETA was dissolved in 6 ml deionised water.

Step 2:- Liquid A was then added to solution B under homogenization at 6000 rpm and the mixture emulsified for 5 min. Solution C was then added dropwise into the emulsion and homogenization was continued for 10 min. The resulting suspension of polyamide capsules, designated "Capsule1X" or "Capsule 1Y" was allowed to age for 24 h to obtain the capsule slurry for use in the following examples.

Note regarding the preparation of Comparative Example AZ:-
[0118]    The preparation method was the same as above except that Perfume raw materials, deisignated "Z" in Table 2 above were used for the preparation of Liquid A.
It was found, however, that the viscosity of Liquid A increased continuously during the mixing process and the final mixture became a gel. A subsequent homogenisation of gelled Liquid A with water solution B was unsuccessful. Thus, the comparative example, which comprised perfume ingredients outwith the scope of the invention, failed.

**Example 2: Methods of evaluating deposition and perfume delivery performance using Capsule 1X and Capsule 1Y**

In the following example, Capsule 1X was used for the study on polyester, whilst Capsule 1Y was used in the cotton study.

**Evaluation of perfume capsule deposition - method**

The delivery of the polyamide perfume capsules, Capsule 1X or Capsule 1Y, to fabric during a model wash procedure was assessed as follows.

1. Surfactant solution was prepared by dissolving LAS (dodecylbenzenesulphonic acid sodium salt) (5.0 g) and Synperonic A7 (5.0 g) in de-ionized water to a total of 1.0 litre. The surfactant concentration of the final solution was 1.0 g/L.

2. Base buffer stock solution was prepared by dissolving sodium carbonate (7.5 g) and sodium bicarbonate (2.4 g) in de-ionized water to a total of 1.0 litre. The base buffer concentration was 0.1 M.

3. 55 ml model wash liquor (1.0 g/L surfactant, 0.01M base buffer) containing 800 ppm polyamide capsule (Capsule 1X or Capsule 1Y) was prepared in a 60 ml bottle and a 5.0 ml aliquot taken out for absorbance recording at 400nm. This absorbance value represents 100% capsules in the wash solution prior to the bottle wash process.

4. One piece (20x20 cm) of non-fluorescent knitted polyester (a total weight of 5.2 g) or three pieces (10x10 cm) of non-fluorescent cotton fabric (total weight 4.5 g) were then placed into the bottle and the bottle sealed.

5. The bottle was then shaken at 125 rpm at 40 °C for 30 min to simulate a main wash procedure.

6. The fabrics were then removed and hand wrung before a 5.0 ml aliquot of the main wash liquor was taken out for absorbance recording at 400 nm. The amount of adsorbed polyamide capsules on the fabric was determined by turbidity difference before/after the step.

7. The bottle was then thoroughly rinsed and the wrung fabrics put in and 50 ml of deionised water added. The bottle was then shaken at 40 °C for 10 minutes at 125 rpm to simulate a rinse procedure.

8. The fabrics were then removed and wrung by hand as before. A 5.0 ml aliquot of the rinse liquor was taken out for absorbance recording at 400 nm. The amount of loss of adsorbed polyamide capsules from fabric in rinse 1 stage could be determined according to turbidity difference. This rinse procedure was repeated and the loss amount of capsules from fabric in this second rinse determined accordingly.

**Evaluation of perfume delivery performance** - **method**

The perfume delivery performance of capsules was assessed using a Tergotometer to simulate top-loading washing machine conditions. The typical procedure was described as following:

Treatment of fabric

- 500 ml deionised water containing liquid detergent (such that the final surfactant concentration in wash liquor was 1.0 g/L,) was added to a 1000 ml pot and Capsule 1X or Capsule 1Y (such that the total amount of perfume was 5 mg) added.
- 5 mg free perfume (X or Y) was added into second pot containing wash liquor, as a control.
- One piece (20 x 20 cm) of non-fluorescent knitted polyester or one piece (20 x 20 cm) of cotton terry towel was then placed into the pot and the wash carried out at 40 °C for 30 min to simulate a main wash procedure.
- The fabrics were then removed and wrung by hand, and the pot thoroughly rinsed.
- The wrung fabric was then put back into the pot and 500 ml of deionised water subsequently added.
- The wash was then continued at 40°C for a further 5 minutes to simulate a rinse procedure.
- The fabrics were then removed and wrung out again.
- The rinse procedure was repeated and the wrung fabric taken out for further evaluation.

Evaluation of perfume delivery

- 1.0 g of fabric was cut from the washed sheet and immersed in 50 ml ethanol in a bottle.
- The remaining fabric was line-dried at room temperature for 24 hr.
- The bottle was then shaken at 40 °C for 24 hr and the perfume extracted from fabric (perfume intensity after wash) evaluated using GC-MS. A further 1.0 g of fabric was cut from the line-dried fabric sheet and the perfume extracted using the same method shown above, and the perfume intensity (after drying) was evaluated utilizing GC-MS. Perfume X was followed by its linalool and limonene components, whilst for Perfume Y, linalyl acetate, ortho-tertiary-butyl cyclohexyl acetate (OTBCA) and cyclopentadecanolid were peaks were picked out.

Note:-

**1. GC-MS conditions for Perfume X evaluation:**

Equipment: Agilent 6890 GC equipped with Agilent 5975B MS and PAL CTC sampler
Injection volume: 1.0 μl
Oven: 50°C hold 1 min, 20°C/min to 180°C, 40°C/min to 280°C and hold for 2 min Run time: 12 min
Inlet: 250°C, splitless
Carrier: He, 1.0 ml/min
Column: HP-Innowax Polyethylene Glycol, Agilent 19091N-133
Acquisition mode: SIM, m/z 68, 93, 136

**2. GC-MS conditions for Perfume Y evaluation:**

Equipment: Agilent 6890 GC equipped with Agilent 5975B MS and PAL CTC sampler
Injection volume: 2.0 μl
Oven: 60°C hold 1 min, 15°C/min to 300°C and hold for 3 min
Run time: 20 min
Inlet: 250°C, splitless
Carrier: He, 1.0 ml/min
Column: HP-5MS, 5% Phenyl Methyl Siloxane, Agilent 19091S-433
Acquisition mode: SIM, m/z 73, 84, 102, 108

**Example 3: Perfume delivery performance of Capsule 1X and Capsule 1Y**

The perfume delivery performance of polyamide perfume Capsule 1X and Capsule 1Y, compared with that of free perfumes X and Y are summarised in Tables 3 and 4.

**Table 3: Perfume delivery performance of polyamide Capsule 1X on polyester, showing Linalool and Limonene components.**

| Perfume ingredient* | Perfume capsule | Perfume intensity on polyester | |
| --- | --- | --- | --- |
| | | After wash | After dry |
| Linalool | Free perfume X (control) | 0.2 | 0.1 |
| | Capsule 1X | 1.2 | 0.4 |
| Limonene | Free perfume X (control) | 5.2 | 1.6 |
| | Capsule 1X | 16.0 | 6.3 |
| * The model perfume for the study is X. Linalool and Limonene was used as model ingredients for assessment. | | | |

**Table 4: Perfume delivery performance of polyamide Capsule 1Y on cotton**

| Perfume ingredient* | Perfume capsule | Perfume intensity on cotton terry towel | |
| --- | --- | --- | --- |
| | | After wash | After dry |
| Linalyl acetate | Free perfume (control) | 1.5 | 0 |
| | polyamide capsule | 5.3 | 0.8 |
| OTBCA | Free perfume (control) | 5.0 | 0.1 |
| | polyamide capsule | 8.1 | 1.8 |

(continued)

| Perfume ingredient* | Perfume capsule | Perfume intensity on cotton terry towel | |
|---|---|---|---|
| | | After wash | After dry |
| Cyclopentadecan olid | Free perfume (control) | 15.0 | 3.4 |
| | polyamide capsule | 33.5 | 12.6 |
| * The model perfume for the study is Y. Linalyl acetate, OTBCA and Cyclotadecanolid were used as model ingredients for assessment as representative for top, middle and base note, respectively. | | | |

It will be seen that the fabric treated with liquid detergent comprising the polyamide capsules in accordance with the invention, had higher perfume intensity compared with that treated with a similar detergent containing the corresponding free perfume.

### Example 4: Long lasting freshness performance using Capsule 1X

The typical procedure was described as follows:

**Capsule 1X** (containing 30mg perfume X) or 30mg free perfume X (control) was dropped on a piece (6x6 cm) of knitted polyester fabric. The fabric was hung and allowed to dry naturally at room temperature. After 7 h and 16 h periods, the fabric was put into 10 ml acetone and sonified for 90 s (circulatory pulse mode including 2 second operating and 5 second suspending) to rupture the perfume capsule completely. The perfume extracted from fabric was evaluated utilizing GC-MS.

The results are shown in **Table 5** below.

**Table 5: Residual perfume on fabric after drying from 7 h and 16 h**

| Perfume ingredient* | Perfume capsule | The residual perfume on the substrate after drying for a certain time | |
|---|---|---|---|
| | | 7h | 16h |
| Linalool | Perfume X (control) | 0 | 0 |
| | Capsule 1X | 29% | 30% |
| Limonene | Perfume X (control) | 0 | 0 |
| | Capsule 1X | 34% | 35% |
| * The model perfume for the study is LBL. | | | |

Linalool and Limonene was used as model ingredients for assessment.

It will be seen that the capsule in accordance with the invention can retain more perfume on the substrate than free perfume. Thus, comparing with free perfume, capsules in accordance with the invention can show better long-lasting freshness.

### Example 5: Comparison between Capsule 2-MC and commercial available Melamine-Formaldehyde based capsule (Capsule A-MC)

Melamine-formaldehyde (MF) based perfume capsules are the current leading technology in the market for long-lasting freshness.

Therefore, a comparison between the polyamide capsules of the present invention with a commercial available MF capsule was carried out.

### Materials

A melamine-formaldehyde capsule, containing a perfume called "Mango Citrus perfume" (size 20 $\mu$m, perfume content is 28%), herein designated "Capsule A-MC", was obtained commercially from Givaudan. The free perfume itself was also obtained from this supplier.

**Preparation of Capsule 2-MC**

A polyamide capsule comprising Mango Citrus perfume (Capsule 2-MC) was prepared. The preparation procedure of Capsule 2-MC is similar as that shown in Example 1. The pH value of Water solution B was adjusted to 7.8 utilizing 1.0 M NaOH.

**Determination of perfume capsule leakage**

**Evaluation procedure for perfume leakage (direct injection method)**

The typical procedure is as follows:-

- 0.375g of Capsule 2-MC slurry was put into a 20 ml vial by burette, to which 3.0 ml of commercially available Persil Small and Mighty (UK) was added. The mixture was then thoroughly mixed.
- The vial was then placed under storage at room temperature for 1 week.
- After the one week storage period, 6 ml of water was added to the vial to reduce the viscosity of the mixture.
- The mixture was then filtrated utilizing a syringe driven filter (0.45 $\mu$m, PES film, ANPEL Scientific Instrument Co., Ltd.) to remove the solid capsules substance.
- The filtrate was further diluted by 4 times by adding acetone and then analyzed using GC-MS (using the following parameters:-

    Equipment: Agilent 6890 GC equipped with Agilent 5975B MS and PAL CTC sampler
    Injection volume: 2.0 $\mu$l
    Oven: 40°C hold 2min, 10°C/min to 200°C, 5°C/min to 220°C and hold for 1min, 10°C/min to 300°C
    Run time: 31min
    Inlet: 280°C, splitless
    Carrier: He, 1.0 ml/min
    Column: HP-5MS, 5% Phenyl Methyl Siloxane, Agilent 19091S-433
    Acquisition mode: SIM, m/z 68, 82, 93, 112, 177, 189, 197, 220

- The amount of perfume that leaked out from capsules to the formulation (denoted as C) could thus be calculated by multiplying perfume intensity from GC-MS result by the total dilution times.

For evaluating the total perfume amount inside the capsules, the following procedure was adopted:-

- The same amount of Capsule 2-MC slurry was transferred into a 20 ml vial and 3 ml of acetone added.

- The vial was then sealed and shaken at 30°C/150 rpm for 3 h and then the capsules in acetone disrupted thoroughly using Sonifier (BRANSON S-250D) for 2 min.

- The resultant acetone solution was evaluated by GC-MS after filtered by a syringe-driven filter (0.45 $\mu$m, Nylon film, ANPEL Scientific Instrument Co., Ltd.) to remove the wall patches of capsule.

- Finally the perfume intensity $C_0$ was obtained and regarded as 100% perfume amount inside the capsules (i.e. no leakage).

The perfume leakage percentage (PLP) can be thus calculated from the formula below:

$$PLP = (C/C0) \times 100\%$$

**Perfume leakage (after 1 week storage) in liquid detergent formulation**

Perfume leakage properties of Capsule 2-MC and Capsule A-MC were compared after being incubated in liquid detergent for 1 week at room temperature and the results shown in **Table 6.**

**Table 6:** Perfume leakage percentage (PLP) from Capsule 2-MC and Capsule A-MC following storage for 1 week.

| Mango Citrus Perfume ingredients | | % leakage from Capsule A-MC | % leakage from Capsule 2-MC |
|---|---|---|---|
| Top note | • -Pinene | 12.7 | 2.4 |
| | Limonene | 20.0 | 3.0 |
| Middle note | Ionone | 36.0 | 17.0 |
| | Lilyaldehyde | 14.3 | 5.0 |
| Base note | Peonile & Nectaryl | 8.2 | 13.0 |

It will be seen that most perfume ingredients showed dramatically lower leakage from Capsule 2-MC compared with that from Capsule A-MC.

### Example 6: Comparison of perfume capsule deposition

The capsule deposition performances of Capsule 2-MC and Capsule A-MC on fabrics were assessed *via* bottle wash process. The typical procedure is similar as that described above in Example 2 except that the starting wash liquor (55 ml) was composed of 3.0 g/L liquid detergent Persil Small and Mighty (UK - commercially available product) and 0.45% Capsule 2-MC (or Capsule A-MC).

The deposition results are given in **Table 7**

**Table 7:** Deposition of Capsule 2-MC and Capsule A-MC during a main wash procedure.

| Capsule type | Formulation | Fabric type | Deposition after main wash (%) | Deposition after twice rinse (%) |
|---|---|---|---|---|
| Capsule A-MC | Persil Small & Mighty | Polyester | 16 | 0 |
| Capsule 2-MC | Persil Small & Mighty | Polyester | 39 | 4 |
| Capsule A-MC | Persil Small & Mighty | Cotton | 38 | 8 |
| Capsule 2-MC | Persil Small & Mighty | Cotton | 51 | 23 |

It will be seen that Capsule 2-MC showed higher deposition performance than Capsule A-MC on both polyester and cotton fabric from both liquid detergent formulations.

### Example 7: Influence of amount (wt %) of long-chain aldehyde in core perfume

### Method of preparation of Capsule

Step 1:- The following liquids were prepared:

**Liquid A:** Tridecylic aldehyde was added into Perfume Y to adjust the amount (wt %) of long chain aldehyde in the resultant perfume. 2.4 ml of each perfume with different amount of long chain aldehyde (shown in Table 8 below) and 0.27 g terephthaloyl chloride were mixed until the terephthaloyl chloride dissolved to obtain an oily liquid.

**Water solution B:** 30 ml deionised water containing 1 wt% PVA (5-88) was prepared.

**Water solution C:** 3.9 ml DETA was dissolved in 6 ml deionised water.

Step 2:- Liquid A was then added to solution B under homogenization at 6000 rpm and the mixture emulsified for 5 min. Solution C was then added dropwise into the emulsion and homogenization was continued for 10 min. The resulting suspension of polyamide capsules was allowed to age for 24 h to obtain the capsule slurry for evaluation.

The capsule slurries were then analysed for aggregation using optical microscopy.

The results are given in Table 8 below.

**Table 8: Influence of amount of long-chain aldehyde in perfume on capsule properties.**

| Amount (wt %) of long chain aldehyde in perfume | 13.4% (Perfume Y) | 15.0% | 20.0% | 100.0% |
|---|---|---|---|---|
| Morphology of polyamide capsule | no aggregation | aggregation | serious aggregation | capsule cannot be prepared |

It will be seen that aggregation begins to appear when the amount of long chain aldehyde is 15 wt % and worsens as the amount is increased to 20 wt %. Use of Perfume Y, however, in accordance with the invention, results in no aggregation.

**Claims**

1. A particle comprising:-

    (a) a core comprising a benefit agent;
    (b) a shell, wherein the shell comprises a polyamide, and wherein the polyamide comprises an aromatic group; and
    (c) an optional deposition aid;

    wherein the benefit agent comprises 70-100 wt %, by total weight of the benefit agent, of a component selected from tertiary alcohols, alpha substituted aldehydes, aliphatic and aromatic ketones and ketenes and mixtures thereof, excluding cyclic aliphatic materials containing polar functional groups;
    and the benefit agent is substantially free from i) aliphatic primary alcohols and ii) aromatic primary alcohols and contains less than 15 wt % of aliphatic aldehydes having a chain length of from 8 to 22, by total weight of the benefit agent.

2. A particle as claimed in claim 1, wherein the deposition aid is a polysaccharide, more preferably a non-ionic polysaccharide.

3. A particle as claimed in claim 1 or claim 2 having an average diameter of from 5 to 50 microns.

4. A particle according to any preceding claim wherein the benefit agent is a fragrance, an antimicrobial compound or a mixture thereof.

5. The particle as claimed in any preceding claim, wherein the polyamide comprises at least one water miscible monomer and one water immiscible organic monomer.

6. The particle as claimed in claim 5, wherein the water miscible monomer comprises a material selected from the group consisting of a diamine, a triamine and mixtures thereof.

7. The particle as claimed in claim 6, wherein the diamines and triamines are selected from the group consisting of diethylene triamine, hexamethylene diamine, ethylene diamine and mixtures thereof.

8. The particle as claimed in claim 5, wherein the water immiscible organic monomer may be selected from the group consisting of diacyl chlorides, triacyl chlorides and mixtures thereof.

9. The particle as claimed in claim 8, wherein the diacyl chlorides is selected from the group consisting of sebacoyl dichloride, adipoyl dichloride, and mixtures thereof.

10. The particle as claimed in claim 8, wherein the triacyl chlorides are selected from the group consisting of terephthaloyl chloride, trimesoyl chloride, acetyl chloride, benzoyl chloride, 1, 3, 5-benzentricarbonyl chloride, and mixtures thereof.

11. A liquid composition comprising the particle as claimed in any preceding claim, which further comprises:-

   a) surfactant selected from anionic, cationic, non-ionic, zwitterionic surfactants; and
   b) solvent, preferably water.

12. A home care or personal care composition comprising at least one particle according to any of claims 1 to 10, the composition preferably being a laundry detergent, laundry conditioner, deodorant, antiperspirant, shampoo, hair conditioner or skin care or skin cleansing product.

13. A composition according to claim 12, which is a deodorant and is preferably in the form of an aerosol, said aerosol being free from ethanol.

14. A method of treatment of a substrate, preferably wherein the substrate is selected from skin, hair and/or textile material, which includes the step of treating the substrate with a composition comprising particles according to any one of claims 1 to 10.

**Patentansprüche**

1. Teilchen, umfassend:

   (a) einen Kern, der ein Vorteilsmittel umfasst,
   (b) eine Schale, wobei die Schale ein Polyamid umfasst und wobei das Polyamid eine aromatische Gruppe umfasst, und
   (c) eine optionale Abscheidungshilfe,

   wobei das Vorteilsmittel 70-100 Gew.-%, bezogen auf das Gesamtgewicht des Vorteilsmittels, an einem Bestandteil umfasst, der aus tertiären Alkoholen, alphasubstituierten Aldehyden, aliphatischen und aromatischen Ketonen und Ketenen und Mischungen davon ausgewählt ist, ausschließlich cyclischer aliphatischer Materialien, die polare funktionelle Gruppen enthalten,
   und wobei das Vorteilsmittel im Wesentlichen frei von i) aliphatischen primären Alkoholen und ii) aromatischen primären Alkoholen ist und weniger als 15 Gew.-% aliphatische Aldehyde einer Kettenlänge von 8 bis 22, bezogen auf das Gewicht des Vorteilsmittels, enthält.

2. Teilchen, wie im Anspruch 1 beansprucht, wobei die Abscheidungshilfe ein Polysaccharid, bevorzugter ein nicht-ionisches Polysaccharid, ist.

3. Teilchen, wie im Anspruch 1 oder 2 beansprucht, das einen durchschnittlichen Durchmesser von 5 bis 50 Mikrometer hat.

4. Teilchen nach irgendeinem vorhergehenden Anspruch, wobei das Vorteilsmittel ein Duftstoff, eine antimikrobielle Verbindung oder eine Mischung davon ist.

5. Teilchen, wie in irgendeinem vorhergehenden Anspruch beansprucht, wobei das Polyamid mindestens ein mit Wasser mischbares Monomer und ein mit Wasser nicht mischbares organisches Monomer umfasst.

6. Teilchen, wie im Anspruch 5 beansprucht, wobei das mit Wasser mischbare Monomer ein Material umfasst, das aus der Gruppe ausgewählt ist, die aus einem Diamin, einem Triamin und Mischungen davon besteht.

7. Teilchen, wie im Anspruch 6 beansprucht, wobei die Diamine und Triamine aus der Gruppe ausgewählt sind, die aus Diethylentriamin, Hexamethylendiamin, Ethylendiamin und Mischungen davon besteht.

8. Teilchen, wie im Anspruch 5 beansprucht, wobei das mit Wasser nicht mischbare organische Monomer aus der Gruppe ausgewählt werden kann, die aus Diacylchloriden, Triacylchloriden und Mischungen davon besteht.

**9.** Teilchen, wie im Anspruch 8 beansprucht, wobei die Diacylchloride aus der Gruppe ausgewählt sind, die aus Sebacoyldichlorid, Adipoyldichlorid und Mischungen davon besteht.

**10.** Teilchen, wie im Anspruch 8 beansprucht, wobei die Triacylchloride aus der Gruppe ausgewählt sind, die aus Terephthaloylchlorid, Trimesoylchlorid, Acetylchlorid, Benzoylchlorid, 1,3,5-Benzoltricarbonylchlorid und Mischungen davon besteht.

**11.** Flüssige Zusammensetzung, die das Teilchen, wie in irgendeinem vorhergehenden Anspruch beansprucht, umfasst, welche ferner umfasst:

> a) Tensid, ausgewählt aus anionischen, kationischen, nicht-ionischen, zwitterionischen Tensiden und
> b) Lösungsmittel, vorzugsweise Wasser.

**12.** Hauspflege- oder Körperpflegezusammensetzung, die mindestens ein Teilchen nach irgendeinem der Ansprüche 1 bis 10 umfasst, wobei die Zusammensetzung vorzugsweise ein Waschmittel, Waschkonditioniermittel, Deodorant, Antitranspirant, Shampoo, ein Haarkonditioniermittel oder Hautpflege- oder Hautreinigungsprodukt darstellt.

**13.** Zusammensetzung nach Anspruch 12, welche ein Deodorant ist und vorzugsweise in Form eines Aerosols vorliegt, wobei das Aerosol frei von Ethanol ist.

**14.** Verfahren zur Behandlung eines Substrats, wobei das Substrat vorzugsweise unter Haut, Haar und/oder textilem Material ausgewählt ist, welches den Schritt der Behandlung des Substrats mit einer Zusammensetzung einbezieht, die Teilchen nach irgendeinem der Ansprüche 1 bis 10 umfasst.

**Revendications**

**1.** Particule comprenant :

> (a) un noyau comprenant un agent bénéfique ;
> (b) une enveloppe, dans laquelle l'enveloppe comprend un polyamide, et dans laquelle le polyamide comprend un groupe aromatique ; et
> (c) un éventuel auxiliaire de dépôt ;

dans laquelle l'agent bénéfique comprend 70-100 % en masse, en masse totale de l'agent bénéfique, d'un constituant choisi parmi des alcools tertiaires, des aldéhydes alpha-substitués, des cétones et cétènes aliphatiques et aromatiques et des mélanges de ceux-ci, excluant des matériaux aliphatiques cycliques contenant des groupes fonctionnels polaires ;
et l'agent bénéfique est pratiquement exempt de i) alcools primaires aliphatiques et ii) alcools primaires aromatiques et contient moins de 15 % en masse d'aldéhydes aliphatiques ayant une longueur de chaîne de 8 à 22, en masse totale de l'agent bénéfique.

**2.** Particule selon la revendication 1, dans laquelle l'auxiliaire de dépôt est un polysaccharide, encore mieux un polysaccharide non-ionique.

**3.** Particule selon la revendication 1 ou la revendication 2 ayant un diamètre moyen de 5 à 50 microns.

**4.** Particule selon l'une quelconque des revendications précédentes dans laquelle l'agent bénéfique est un parfum, un composé antimicrobien ou un mélange de ceux-ci.

**5.** Particule selon l'une quelconque des revendications précédentes, dans laquelle le polyamide comprend au moins un monomère miscible à l'eau et un monomère organique immiscible à l'eau.

**6.** Particule selon la revendication 5, dans laquelle le monomère miscible à l'eau comprend un matériau choisi dans le groupe consistant en une diamine, une triamine et des mélanges de celles-ci.

**7.** Particule selon la revendication 6, dans laquelle les diamines et triamines sont choisies dans le groupe consistant en diéthylène triamine, hexaméthylène diamine, éthylène diamine et mélanges de celles-ci.

**8.** Particule selon la revendication 5, dans laquelle le monomère organique immiscible à l'eau peut être choisi dans le groupe consistant en chlorures de diacyle, chlorures de triacyle et mélanges de ceux-ci.

**9.** Particule selon la revendication 8, dans laquelle les chlorures de diacyle sont choisis dans le groupe consistant en dichlorure de sébacoyle, dichlorure d'adipoyle, et mélanges de ceux-ci.

**10.** Particule selon la revendication 8, dans laquelle les chlorures de triacyle sont choisis dans le groupe consistant en chlorure de téréphtaloyle, chlorure de trimésoyle, chlorure d'acétyle, chlorure de benzoyle, chlorure de 1,3,5-benzènetricarbonyle, et mélanges de ceux-ci.

**11.** Composition liquide comprenant la particule selon l'une quelconque des revendications précédentes, laquelle comprend de plus :

a) un tensioactif choisi parmi des tensioactifs anioniques, cationiques, non-ioniques, zwitterioniques ; et
b) un solvant, de préférence de l'eau.

**12.** Composition pour le soin de la maison ou le soin de la personne comprenant au moins une particule selon l'une quelconque des revendications 1 à 10, la composition étant de préférence un détergent de lessive, un conditionneur de lessive, un déodorant, un antiperspirant, un shampoing, un après-shampoing ou un produit pour le soin de la peau ou pour le nettoyage de la peau.

**13.** Composition selon la revendication 12, laquelle est un déodorant et est de préférence dans la forme d'un aérosol, ledit aérosol étant exempt d'éthanol.

**14.** Procédé de traitement d'un substrat, de préférence dans lequel le substrat est choisi parmi la peau, les cheveux et/ou un matériau textile, qui comprend l'étape de traitement du substrat avec une composition comprenant des particules selon l'une quelconque des revendications 1 à 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 12085864 A
- US 4145184 A
- WO 09047745 A
- WO 11056904 A
- WO 10105922 A
- EP 1640063 A
- WO 11161265 A
- US 4702857 A
- US 4968451 A
- US 4711730 A
- US 4721580 A
- US 4877896 A
- US 3915867 A
- GB 1437950 A
- GB 1473201 A
- GB 1473202 A
- GB 1470250 A
- EP 164514 B
- GB 1429143 A
- EP 384070 A
- GB 2123044 B
- US 4751015 A
- US 4818426 A
- EP 402971 A
- EP 284292 A
- EP 303520 A
- US 4686063 A
- US 5397501 A
- EP 325288 A
- EP 349940 A
- DE 3823172
- EP 325289 A
- EP 458397 A
- EP 458398 A
- EP 509787 A
- GB 1243785 A
- EP 340013 A
- EP 367339 A
- EP 390251 A
- EP 420317 A

### Non-patent literature cited in the description

- Fenaroli's Handbook of Flavour Ingredients. CRC Press, 1975
- **M. B. JACOBS.** Synthetic Food Adjuncts. 1947
- **S. ARCTANDER.** *Perfume and Flavour Chemicals,* 1969
- **POUCHER.** *Journal of the Society of Cosmetic Chemists,* 1955, vol. 6 (2), 80
- Surfactants Science Series. 1990, vol. 34
- SURFACTANTS SCIENCE SERIES. 1991, vol. 37
- SURFACTANTS SCIENCE SERIES. Marcel Dekker Inc, 1994, vol. 53
- **D MARSH.** Handbook of Lipid Bilayers. CRC Press, 1990, 137, , 337